# EUROPEAN PATENT APPLICATION

(11) **EP 1 764 055 A1**
(43) Date of publication of application: **21.03.2007**
(21) Application number: 05108584.3
(22) Date of filing: 19.09.2005
(51) Int. Cl.: A61B 17/92, A61B 17/88

(54) **Device for removal of fastening means from human tissue**

(71) Applicant: Inion Ltd., 33520 Tampere (FI)
(72) Inventor: Happonen, Harri, 33820, TAMPERE (FI); Koho, Petri, 33710, Tampere (FI)
(74) Representative: Kaukonen, Juha Veikko

(57) **Abstract**

A device for removal of fastening means from human tissue. The device comprises a shaft (2) having proximal and distal ends (3, 4) and a longitudinal centre line (C). The distal end (4) comprises a thread section (6) having a centre line (D) that is coaxial with the longitudinal centre line (C) of the shaft (2) and a left-hand thread (7) that screws itself around the centre line (D), and a counter surface (8a, 8b) that is disposed outside the thread (7) and has a surface component direction which intersects one of said centre lines (C, D). The counter surface (8a, 8b) is disposed so as to make contact with the fastening means to be removed when the thread (7) is rotated into a hole in the fastening means.

## Description

### FIELD OF THE INVENTION

The invention relates to a device for removal of fastening means from human tissue, and more particularly to a device for removal of fastening means from bone tissue.

### BACKGROUND OF THE INVENTION

Use of biodegradable materials or materials that dissolve in the human body is increasing in the manufacture of fastening means used in surgery.

Fastening means, such as screws, pins, anchors, rivets, tacks etc. made of biodegradable materials have the benefit that they dissolve within the human body and, therefore, there is no need to remove the fastening means from the human body in another operation. It should be noted that all materials that dissolve in the human body are below referred to as 'biodegradable'.

It sometimes happens for one reason or another that a fastening means, for example a screw, breaks during its installation so that a part of the screw remains in a hole into which it was screwed. It is also possible that a head portion of a screw becomes damaged so that the screw cannot be screwed in its intended position in the hole, nor unscrewed out of the hole in order to remove the screw. Furthermore, a fastening means can be broken due to excessive loading or accidents e.g. due to shearing forces, at the fixation interface. Usually, a broken or damaged fastening means needs to be removed from the human body. It is also possible that a fastening means which is not broken or damaged is removed from the human body.

Some manufacturers have provided various retraction tools for removing a broken or damaged screw from bone tissue.

US 5,697,935 discloses an apparatus that comprises a hollow boring drill. The drill has an internal cavity and an opening in its distal end to said cavity. The distal end comprises also a cutting surface around the opening. The operator places the cutting surface against bone tissue around the fastening means to be removed, and rotates the drill so as to cut into the bone tissue around the fastening means. The surface of the internal cavity bites and adheres to the external surface of the fastening means. The operator then can move the drill away from the bone tissue and remove the fastening means from the bone tissue.

One of the problems associated with the above apparatus and its use is that a fair quantity of bone tissue needs to be removed during the removal process.

US 6,004,321 discloses an apparatus that is provided for retraction of cannulated screws from bone tissue. The apparatus comprises a reverse, self-tapping helix on a conical surface and a feeler shaft connected to the tip of the conical surface. The feeler shaft guides the apparatus at the entry to the cannula of a fractured screw, and the reverse helix bites to the material of the screw to be removed.

One of the problems associated with the above apparatus is that the reverse helix on a conical surface can expand the biodegradable material, in which case the screw tends to adhere even more to the hole. Consequently, the screw breaks into pieces.

### BRIEF DESCRIPTION OF THE INVENTION

An object of the present invention is to provide a device so as to alleviate the above disadvantages. The objects of the invention are achieved by a device wherein the distal end comprises a thread section having a centre line that is coaxial with the longitudinal centre line of the shaft, and a left hand thread that screws around the centre line, and a counter surface that is disposed outside the thread and having a surface component direction which intersects one of said centre lines, the counter surface being disposed so that it can make contact with the fastening means to be removed as the thread is rotated into a hole in the fastening means.

The invention is based on the idea of using a combination of a left-hand screw and a counter surface in order to remove of a fastening means.

An advantage of the device of the invention is that a broken or damaged fastening means can be removed without damaging or expanding the fixation hole where the fastening means is fastened.

### BRIEF DESCRIPTION OF THE DRAWINGS

In the following, the invention will be described in greater detail by means of preferred embodiments and with reference to the accompanying drawings, in which
Figure 1 is a schematic side view of a device according to the invention,
Figure 2 is a schematic side view of a detail of the device illustrated in Figure 1,
Figure 3 is a schematic and partly cross-sectional side view of a second device according to the invention arranged in a fastening means to be removed from bone tissue,
Figure 4 is a schematic and partly cross-sectional side view of a third device according to the invention arranged in a fastening means to be removed from bone tissue, and
Figures 5a to 5e are schematic cross-sectional side views of some other thread designs of devices according to the invention.

For the sake of clarity, the figures show the invention in a simplified manner. Like reference numbers identify like elements.

### DETAILED DESCRIPTION OF THE INVENTION

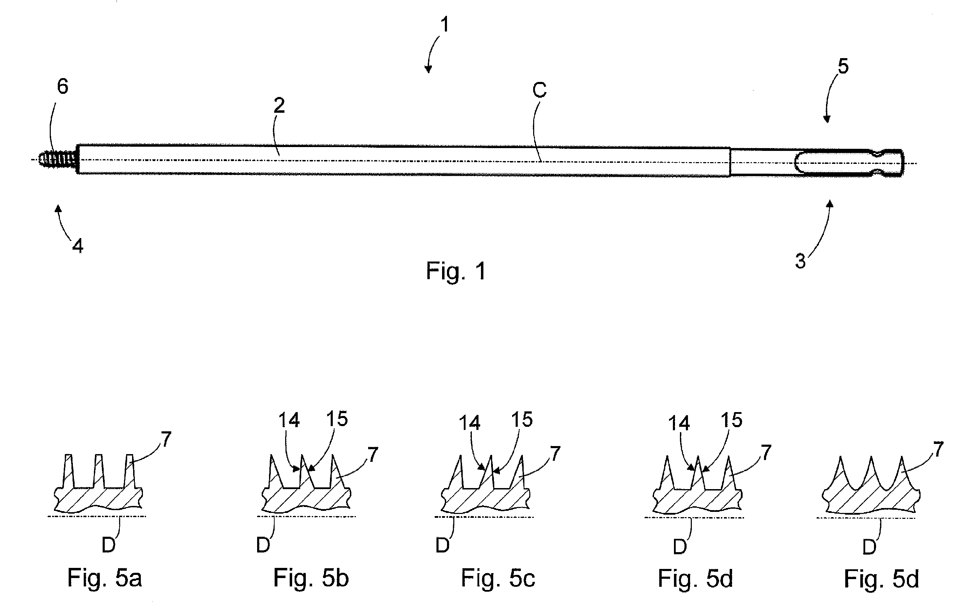

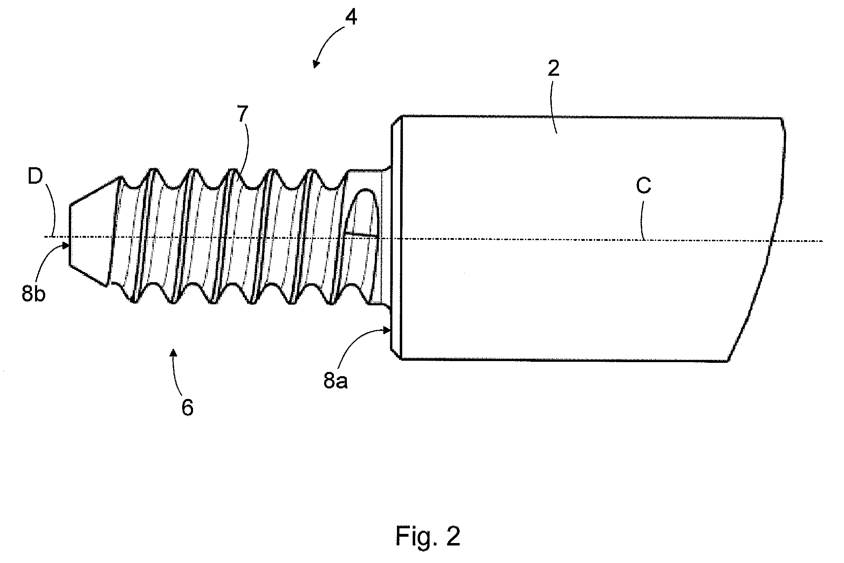

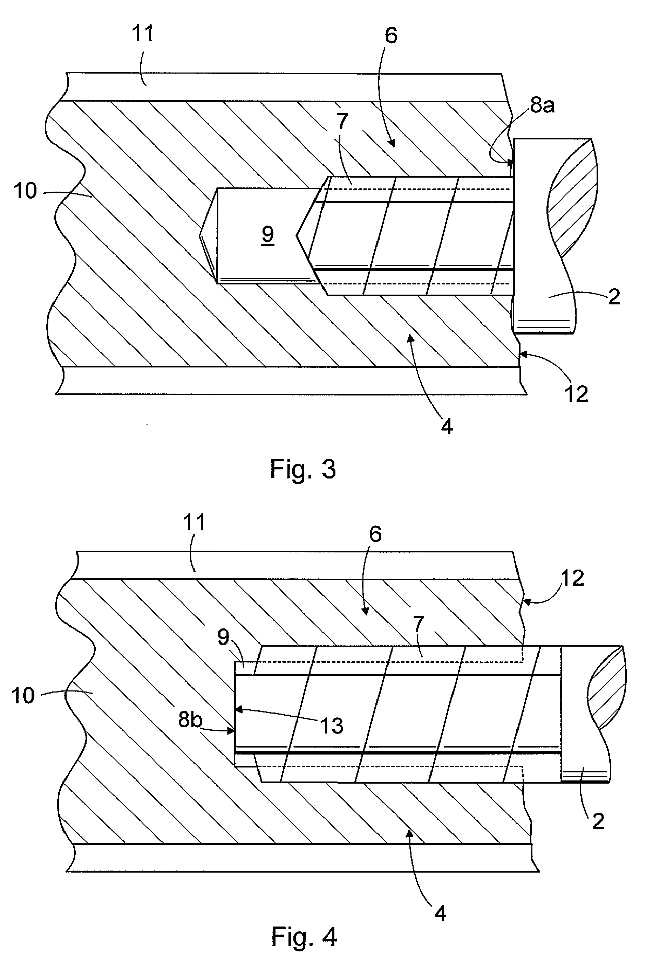

Figure 1 is a schematic side view of a device according to the invention, and Figure 2 is a schematic side view of a detail of the same device.

The device comprises a shaft 2 made e.g. of stainless steel or other similar material that can be used in the manufacture of surgical devices and tools. The shaft 2 has an elongated shape having a proximal end 3 and a distal end 4.

In the proximal end are provided fixing means 5 for fixing the device 1 in a handle or a tool by means of which the device can be rotated around a centre line C of the shaft 2. The tool can be e.g. a drilling machine or a gimlet or some other suitable tool. The fixing means 5 can, of course, be designed in a manner different to that shown in Figure 1. As an alternative, the proximal end 3 can also include a handle that is attached to or integrated in the shaft 2.

It is to be noted that the distance of the proximal end 3 and the distal end 4 can also be considerably shorter than that shown in Figure 1.

The distal end 4 is provided with a thread section 6. As shown in Figure 2, the thread section 6 has a centre line D that is coaxial with the longitudinal centre line C of the shaft 2.

The threaded section 6 is an integral part of the shaft 2 in the device shown in Figure 1. Alternatively, the threaded section 6 can be detachably attached to the shaft 2 by attaching means, e.g. threads.

The thread section 6 includes an external screw thread 7 that screws itself around the centre line D of the thread section 6. The screw thread 7 is a left-hand thread, i.e. opposite to usual threads. Therefore, it must be rotated anti-clockwise in order to screw the thread 7 into a hole.

One screw thread 7 is provided in the thread section 6, extending as a uniform thread from one end to the other end of the thread section 6. The outer diameter of the screw thread 7 is constant, with the exception of a convergent area close to the outmost end of the distal end 4. The height of the screw thread 7 is constant, apart from the convergent area. The height of the screw thread is about 15% of the outer diameter of the thread in the thread section shown in Figure 2. The height of the screw thread 7 may be advantageously selected from a range between 5% and 20%. Such a relatively high thread is preferable in order to prevent the fastening means from expanding in its hole. For the same reason, a relatively thin thread is also preferable.

A pitch of the screw thread 7 is constant throughout the length of the thread section 6.

The distal end 4 is also provided with a first counter surface 8a and a second counter surface 8b. The first counter surface 8a is located between the thread section 6 and the shaft 2. It is a collar-like plane surface perpendicular to the centre line D of the thread section 6. The outer diameter of the first counter surface 8a is substantially larger than that of threaded section 6 and equal to the diameter of the shaft 2. Alternatively, the outer diameter of the first counter surface 8a can be smaller or larger than the diameter of the shaft 2.

The second counter surface 8b is arranged in the outmost end of the distal end 4 of the device 1, at the end of the thread section 6.

Both of the counter surfaces 8a, 8b have a surface component that intersects the centre line of the thread section 6. More accurately, the counter surfaces 8a, 8b are plane surfaces that are arranged perpendicularly to the centre line D of the thread section 6. Alternatively, the first counter surface 8a may resemble a part of a doughnut surface, or a blunt cone surface, for instance, but, nevertheless, the embodiment shown in Figure 2 is usually preferable.

The second counter surface 8b may also resemble a blunt cone or a part of a spherical surface, for instance.

The finish of the counter surfaces 8a, 8b may be roughened up or smoothed, for instance, preferably slightly roughened.

Figure 3 is a schematic and partly cross-sectional side view of a second device according to the invention arranged in a fastening means to be removed from bone tissue. It is to be noted that only the distal end 4 of the device is shown in Figure 3.

The distal end 4 is screwed into a borehole 9 that is made in a fastening means 10 to be removed from bone tissue. The fastening means 10 is a screw made of a biodegradable material. Said bone tissue is not shown in Figure 3, but the screw 10 is fastened to a hole in it by a thread 11. The thread 11 is a right-hand thread, i.e. the screw penetrates into the hole when turned clockwise, and respectively, unscrews out of the hole when turned anti-clockwise.

The screw 10 has fractured along a surface depicted by reference number 12. The part of the screw 10 shown in Figure 3, or at least its thread 11, is jammed in the bone tissue. A jamming-force interacts between the screw 10 and the bone tissue and keeps the screw 10 jammed in the bone tissue.

The device of the invention can be used as follows.

The operator has first drilled the borehole 9 in the screw 10. The borehole 9 is preferably made parallel to the longitudinal axis of the screw 10. The borehole is also preferably coaxial with said longitudinal axis. A slight non-paralellism or offset does not cause any harm to the operation.

The borehole 9 can be made with any known drill bit and drilling method and, therefore, this is not discussed in more detail herein. The diameter of the drill bit should, of course, match the dimensions of the thread 7. Especially, the outer diameter of the drill bit is preferably slightly larger than the inner diameter of the thread 7. In this way, the threaded section 6 does not expand radially the screw to be removed.

When the borehole 9 has been finished, the operator takes the drill bit out of the borehole 9. Next, the operator guides the distal end 4 to the open end of the borehole 9, and begins to rotate the thread section 6 into the borehole 9 around centre line D of the thread section. The direction of the rotation is anti-clockwise. The thread 7 of the thread section 6 bites into the wall of the borehole 9 and the thread section 6 penetrates into the borehole 9.

As the operator continues the rotation of the tool, the counter surface 8a is squeezed against a fracture surface 12. This very situation is shown in Figure 3. The operator continues to rotate the device, whereupon the thread 7 of the thread section 6 pulls the counter surface 8a and the fracture surface 12 against each other by a specific pulling force. Due to the pulling force, a frictional force is provided between the counter surface 8a and the fracture surface 12. Another frictional force is also provided between the thread section 6 and the borehole 9. The screw 10 begins to rotate anti-clockwise with the device as a sum of frictional forces between the counter surface 8a and the fracture surface 12, and, on the other hand, between the thread section 6 and the borehole exceeds the above-mentioned jamming force. Thus the screw 10 can be unscrewed out of the borehole 9. Ideally, the screw 10 remains in the thread section 6 until the operator removes it therefrom. It is also possible that the fastening means begins to rotate with the device, i.e. unscrew, before the counter surface 8a squeezes against the fastening means.

It is to be noted that the device is not only suitable for removal of broken fastening means but it is also suitable for removal of fastening means whose head is damaged as well as fastening means not damaged at all but which still need to be removed from a human or animal body. The device is suitable for removal of any fastening means made of a biodegradable material or even fastening means made of any biostabile polymer material into which the threaded section can be attached that needs to be removed from a human or animal body.

The device is also suitable for removal of cannulated fastening means. In this case, it is not always necessary to drill a borehole in the fastening means prior to insertion of the thread section 6. Instead, the cannula of the fastening means can be exploited as a hole where the thread section 6 is inserted in.

It is to be noted that the term "fastening means" refers to screws, pins, pegs, anchors, rivets, tacks etc. For example, if a need exists for removal of a pin that is not fixed to tissue with any screw thread, the pin may still be screwed out of its attachment hole. Alternatively, as the thread section 6 is attached firmly to the pin, the pin is simply pulled out of the hole with the help of the device.

It is also to be noted that the first counter surface 8a shown in Figure 2 functions as disclosed above if it is the first counter surface of the two counter surfaces 8a, 8b that squeezes against the fastening means to be removed.

An embodiment of the device of the invention has a counter surface 8a arranged between a thread section 6 and a shaft 2, i.e. similarly to the embodiment shown in Figure 3. In addition a drill bit is provided that is disposed at the outmost end of the distal end. The drill bit has a left-hand cutting edge. Such a device first cuts a borehole in a fastening means to be removed and then bites to said borehole with the thread section, all being provided within the same device and with the same rotation movement.

Figure 4 is a schematic and partly cross-sectional side view of a third device according to the invention arranged in a fastening means to be removed from bone tissue. The device comprises a shaft 2, shown only in part, and a distal end having a threaded section 6 with a left-hand screw thread 7. The distal end 4 also comprises a counter surface 8b fitted at the outmost end of the distal end 4.

Basically, the device shown in Figure 4 functions in the same way as the device shown in Figure 3. The main difference is that because the counter surface 8b is situated at the outmost end of the distal end 4, it squeezes against a bottom 13 of the borehole 9. Accordingly, the thread 7 of the thread section 6 pushes the counter surface 8b against the bottom 13 of the borehole 9, and as soon as a sum of frictional forces between the counter surface 8b and the bottom 13 of the borehole, and, on the other hand, between the thread section 6 and the borehole 9 exceeds the jamming force of the fastening means 10, which is a screw in Figure 4, the fastening means 10 begins to screw itself out of the tissue.

It is to be noted that the second counter surface 8b shown in Figure 2 functions as discussed above if it is the first counter surface of the two counter surfaces 8a, 8b that squeezes against the fastening means to be removed.

Figures 5a to 5e are schematic cross-sectional side views of some other thread designs of devices according to the invention. The thread 7 of the thread section 6 can be realized in various ways. Figures 5a to 5e show some examples of this. In Figure 5a the thread 7 is a trapezoid thread.

In figure 5b, the angle between a leading face 14 of the thread 7 and a plane perpendicular to the centre line D of the threaded section is smaller than the angle of a back face 15 of the thread 7. Contrary to this, the angle between the leading face 14 of the thread 7 and a plane perpendicular to the centre line D of the thread section is larger than the angle of the back face 15 of the thread 7 in Figure 5c. In Figure 5d, the leading face 14 and the back face 15 are arranged into a very same angle with that of a plane perpendicular to the centre line D. In Figure 5d, the profile of the thread 7 has a sharp outer edge and curved leading and back faces.

The thread section 6 may also have two or more screw threads 7, in which case it is a multi-end threaded section. The screw thread 7 can be discontinuous such that at one or more points, it is completely or partially cut. In addition, the profile, pitch, height and thickness of the screw thread 7 may vary throughout the length of the threaded section. It will be obvious to a person skilled in the art that as technology advances, the inventive concept can be implemented in various ways. The invention and its embodiments are not limited to the examples described above but may vary within the scope of the claims.

## Claims

1. A device for removal of fastening means from human tissue, the device comprising
a shaft (2) having proximal and distal ends (3, 4) and a longitudinal centre line (C), **characterized in that**
the distal end (4) comprises a thread section (6) having a centre line (D) that is coaxial with the longitudinal centre line (C) of the shaft (2) and
a left-hand thread (7) that screws itself around the centre line (D), and
a counter surface (8a, 8b) that is disposed outside the thread (7) and has a surface component direction which intersects one of said centre lines (C, D),
the counter surface (8a, 8b) is disposed so as to make contact with the fastening means to be removed when the thread (7) is rotated into a hole in the fastening means.

2. A device as claimed in claim 1, **characterized in that** the thread (7) is fitted between the counter surface (8a) and an outmost end of the distal end of the device.

3. A device as claimed in claim 1, **characterized in that** the counter surface (8b) is fitted at the outmost end of the distal end.

4. A device as claimed in claim 1, **characterized in that** it comprises two counter surfaces: a first counter surface (8a) arranged such that the thread (7) is located between it and the outmost end of the distal end, and a second counter surface (8b) fitted at the outmost end of the distal end.

5. A device as claimed in any one of claims 1 to 4, **characterized in that** an outer diameter of the thread (7) is invariable substantially throughout its length.

6. A device as claimed in any one of the preceding claims, **characterized in that** a pitch of the thread (7) is constant throughout its length.

7. A device as claimed in claim 1, **characterized in that** the thread (7) is fitted between the counter surface (8a) and the outmost end of the distal end (4) of the device, and a drill bit is disposed at the outmost end of the distal end, the drill bit having a left-hand cutting edge.
